# EUROPEAN PATENT SPECIFICATION

(11) **EP 1 562 626 B1**
(45) Date of publication and mention of the grant of the patent: **28.11.2007**
(21) Application number: 03779390.8
(22) Date of filing: 28.10.2003
(51) Int. Cl.: A61K 38/16

(54) **STABILIZED, SOLID-STATE POLYPEPTIDE PARTICLES**
STABILISIERTE FESTE POLYPEPTIDPARTIKEL
PARTICULES POLYPEPTIDIQUES SOLIDES STABILISEES

(30) Priority: 29.10.2002 US 422289 P
(43) Date of publication of application: 17.08.2005
(73) Proprietor: ALZA CORPORATION, Mountain View, CA 94039-7210 (US)
(72) Inventor: BENTZ, Johanna, Newark, CA 94560 (US); KANG, Ling-Ling, Palo Alto, CA 94306 (US)
(74) Representative: Williams, Paul Edwin
(86) International application number: PCT/US2003/034229
(87) International publication number: WO 2004/039392

(56) References cited:
- EP-A- 0 790 650
- WO-A-03/068805
- WO-A-03/080108
- US-A- 5 874 408
- DATABASE WPI Section Ch, Week 199041 Derwent Publications Ltd., London, GB; Class B04, AN 1990-309038 XP002277579 & JP 02 218618 A (GREEN CROSS CORP) 31 August 1990 (1990-08-31)

## Description

The invention is directed to polypeptide formulations in solid-state, wherein the polypeptide is stabilized against degradation at elevated temperatures over an extended period of time. Also described are formulations and methods for preparing solid-state polypeptide particles, wherein the polypeptides are stabilized through one or more stabilizing conditions.

Implantable devices capable of delivering desired doses of an active agent, such as a polypeptide, over extended periods of time are known in the art. For example, U.S. patents 5,728,396, 5,985,305, 6,113,938, 6,156,331, 6,375,978, and 6,395,292 teach implantable osmotic devices capable of delivering a stable active agent formulation, such as a solution or a suspension, and at a desired rate over an extended period of time (*i.e.,* a period ranging from about two weeks to several months or more). Implantable drug delivery systems also include depot-type materials, such as those described in U.S. patents 6,468,961, 6,331,311, and 6,130,200. Depot materials typically sequester the active agent within a biodegradable or bioerodible depot material such that the active agent is delivered from the implanted depot material based on diffusion of the active agent or degradation or erosion of the depot material. Exemplary depot materials include PLGA-based systems, which are typically capable of delivering the active agent over periods ranging from about 2 weeks to 6 months. Because they can be designed to deliver a desired active agent at therapeutic levels over an extended period of time, implantable delivery systems can advantageously provide long-term therapeutic dosing of a desired active agent without requiring frequent visits to a healthcare provider or repetitive self-medication. However, delivering therapeutic polypeptides over an extended period of time using an implantable drug delivery system presents various technical challenges.

In particular, it has proven challenging to maintain the stability of therapeutic polypeptides loaded in an implantable delivery system designed to deliver the polypeptide over a period of weeks or months. In order to achieve an implantable system of suitable size that provides delivery of therapeutic doses of a polypeptide over an extended period of time, it is generally necessary to load the system with a solution or suspension containing a high concentration of the polypeptide to be delivered. However, if such a solution or suspension is exposed to temperatures that approach or exceed physiological conditions (e.g., temperatures that approach or exceed 37° C) over an extended period of time, the polypeptide contained in the solution or suspension will degrade if the polypeptide is not stabilized. Degradation of polypeptides exposed to temperatures that approximate or exceed physiological conditions can proceed through various pathways and can alter, reduce or destroy the biological activity of the polypeptide. Therefore, in order to achieve an implantable delivery system capable of successfully delivering a therapeutic polypeptide over an extended period of time, the polypeptide loaded therein must be stabilized against degradation such that the system is capable of delivering therapeutic doses of biologically active polypeptide over the functional life of the implantable system.

Sugars have been used in polypeptide formulations to stabilize the polypeptides contained therein against degradation over time. In particular, sugars have been used as lyoprotectants, working to inhibit polypeptide aggregation by reducing molecular unfolding during the lyophilization process. Sugars also impart long-term stability by limiting molecular mobility and by minimizing molecular interactions during and after the lyophilization process. However, when using a sugar to stabilize a polypeptide, it is often necessary to use large amounts in order to achieve a desired degree of stabilization. As is taught in U.S. patent 6,267,958 to Andya et al., as much as 100 to 510 molar ratios of stabilizing sugar may be needed in order to achieve an acceptable stabilization effect, and polypeptide formulations including such large amounts of stabilizing sugar are not well suited for loading in an implantable drug delivery system.

The abstract of JP 02 218 618 A discloses a powder prepared by lyophilising an aqueous solution containing thrombin at pH 5.6-7.6 and a stabilising saccharide which solution has been heated at 60 °C for 5-15 hours. The powder is mixed with lactose as a vehicle and is used as a hemostatic remedy.

EP 0 791 650 A discloses aqueous solutions containing a reverse transcriptase stabilized by trehalose. The preferred pH of the reverse transcriptase composition is in the range from 5 to 10, more preferably from 6 to 9. The aqueous solutions may be freeze-dried for improved storage stability by mixing with a buffer solution then subjecting to freeze-drying. Freeze-dried compositions prepared using aqueous solutions comprising a reverse transcriptase and trehalose are disclosed.

US 5 874 408 A discloses a lyophilized formulation for recombinant Factor VIII suitable for treating hemophilia comprising recombinant Factor VIII and sucrose. The document mentions that other sugars (including trehalose) may be included to contribute to the crystalline component of the formulation.

Where a high concentration of stabilizing sugar is needed to achieve a desired degree of polypeptide stabilization within a delivery system, the overall bulk of the polypeptide formulation contained within the system increases, while the maximum concentration of polypeptide that can be loaded into the system decreases. As the concentration of the polypeptide in the formulation loaded into an implantable system decreases, the amount of polypeptide formulation required to achieve a desired dosing regimen and the minimum size of the implanted system increase. It would be an improvement in the art, therefore, to provide formulations and methods for stabilizing therapeutic polypeptides that reduce or altogether eliminate the need for a stabilizing sugar. It would be a further improvement in the art if such formulations and methods were capable of stabilizing polypeptides to such an extent that the polypeptides could be loaded into an implantable delivery system at a high concentration and still exhibit acceptable stability and therapeutic activity after exposure to temperatures up to or exceeding physiological conditions over an extended period of time.

The present application concerns solid-state polypeptide particles, wherein the polypeptide contained within the particles is stabilized against degradation at temperatures up to and exceeding physiological conditions. As they are used herein, the term "physiological conditions" refers to environments having a temperature of about 37° C and the term "polypeptide" includes oligopeptides and proteins and encompasses any natural or synthetic compound containing two or more amino acids linked by the carboxyl group of one amino acid to the amino group of another. The polypeptide particles described herein incorporate a polypeptide material that is stabilized against degradation by one or more stabilizing conditions. In particular, the polypeptide particles described herein are formulated to stabilize the polypeptide contained therein by controlling one or more of the following particle conditions: pH, sugar content, surfactant content, buffer content, and metal ion concentration. Because the polypeptide particles can be formulated to combine the additive effects of two or more stabilizing conditions, where the polypeptide particles include a stabilizing sugar, the amount of stabilizing sugar needed to achieve acceptable polypeptide stability is significantly reduced. The polypeptide particles may be formulated to stabilize the polypeptide contained therein without the use of a stabilizing sugar.

The present application also describes aqueous stabilizing solutions. In order to form the solid-state polypeptide particles, an aqueous stabilizing solution, which contains the polypeptide to be stabilized, may be formed and subjected to a suitable particle formation process, such as a lyophilization or spray drying process. An aqueous stabilizing solution, therefore, is formulated such that, upon subjecting the aqueous stabilizing solution to a particle formation process, the aqueous stabilizing solution yields solid-state polypeptide particles that contain polypeptide material stabilized by one or more stabilizing conditions. By controlling the pH, sugar content, surfactant content, buffer content, metal ion concentration, or polypeptide concentration of an aqueous stabilizing solution, solid-state polypeptide particles having a wide range of desired stabilization characteristics can be produced.

The present application further describes methods for producing stabilized, solid-state polypeptide particles. The method includes dissolving the polypeptide to be stabilized in an aqueous stabilizing solution and reconstituting the polypeptide in solid-state polypeptide particles. The formulation of the solid-state peptide particles produced by the method will depend on the aqueous stabilizing solution used. In one example, the method includes dissolving a polypeptide to be stabilized in an acidic stabilizing solution. In another example, the method includes dissolving a polypeptide to be stabilized in an acidic stabilizing solution in the presence of a stabilizing sugar, a metal ion, or both a stabilizing sugar and a metal ion. In yet another example, the method includes dissolving a polypeptide to be stabilized in a buffered, near neutral stabilizing solution in the presence of a surfactant, a stabilizing sugar, a metal ion, or both a stabilizing sugar and a metal ion. In each example of the method, the step of reconstituting solid-state peptide particles may be carried out by subjecting the aqueous stabilizing solution to a suitable particle formation process, such as a lyophilization or spray drying process. Though the method may be varied to achieve particles having any one of a variety of formulations, in each instance the method is tailored to provide solid-state polypeptide particles, wherein the polypeptide is stabilized through one or more stabilizing conditions.

The solid-state polypeptide particles described herein provide excellent polypeptide stabilization, allowing recovery of up to 96% of the stabilized peptide after two months storage at 60° C. Moreover, the solid-state polypeptide particles can be loaded into suspension formulations at relatively high concentrations (i.e., 25% polypeptide particle or more), which facilitates the formulation of suspensions having relatively higher concentrations of the stabilized polypeptide. The solid-state polypeptide particles, therefore, facilitate the loading of an acceptably-sized implant delivery system with a concentration of stabilized, therapeutic polypeptide that is sufficiently high to enable delivery of therapeutic doses of the stabilized, therapeutic polypeptide over an extended period of time.

FIG. 1 provides a graph illustrating the total degradation (RP-HPLC) and Aggregation (SEC) of lyophilized PACAP (ammonium acetate, app. pH 6.4) upon storage at 40°C for 3 months.

FIG. 2 provides a graph illustrating the stability of lyophilized PACAP stored at 4°C, 40°C and 60°C for 3 Months.

FIG. 3 provides graph illustrating the suppression of PACAP aggregate formation at 40°C where PACAP is formulated in solid-state particles using various excipients.

FIG. 4 provides graph illustrating the suppression of PACAP aggregate formation at 60°C where PACAP is formulated in solid-state particles using various excipients.

FIG. 5 provides a graph illustrating the stabilization effect of Histidine, Succinate, and Sucrose on total PACAP degradation at 40°C.

FIG. 6 provides a graph illustrating the stabilization effect of sucrose on suppression of PACAP aggregate formation in BA/PVP Suspension at 40°C.

FIG. 7 provides a graph illustrating the stabilization effect of sucrose on suppression of PACAP aggregate formation in LL/GML/PVP Suspension at 40°C.

FIG. 8 provides a graph illustrating the total Degradation of PACAP in different suspension vehicles upon storage at 40°C for 3 months.

FIG. 9 illustrates the total degradation, as determined by RP HPLC, and aggregation, as determined by SEC, of PACAP contained within particles lyophilized at an apparent pH of 2, an apparent pH of 4, and an apparent pH of 6 upon storage at 60°C for 2 months.

Table 1 sets forth the results of a study conducted to evaluate the stability of PACAP dispersed in various suspension vehicles upon incubating the PACAP suspensions at 65°C for 4 hrs.

Table 2 sets forth the results of a study conducted to evaluate the stability PACAP incubated at 37°C for over a period of 17 days, wherein the PACAP evaluated was lyophilized PACAP alone or PACAP dispersed within one of three different suspension vehicles.

Table 3 sets forth the results of a study conducted to evaluate the stability of PACAP incubated at 60°C over a period of 17 days, wherein the PACAP evaluated was lyophilized PACAP alone or PACAP dispersed within one of three different suspension vehicles.

Table 4 provides the estimated degradation and aggregation rates of PACAP where PACAP is incorporated in solid-state particles including various different excipients and such particles are incubated at 40°C and 60°C.
Table 5 provides the estimated degradation and aggregation rates of PACAP contained within solid-state particles formed from three different solutions prepared in NH₄OAc buffer at pH6, with each of the three solutions containing a different amount of sucrose.
Table 6 sets forth the results of a study conducted to evaluate the stabilization of PACAP achieved by formulating solid state solid-state PACAP particles at varying pH and with one of three different sugars.
Table 7 sets forth the results of a study conducted to evaluate the additive stabilization of PACAP achieved by formulating solid-state PACAP particles at varying pH and with one or more different excipients.
Table 8 sets forth the results of a study conducted to evaluate the stability of PACAP achieved by formulating solid-state PACAP particles at varying pH with CaCl₂, with Histidine, and with both CaCl₂ and Histidine.

According to an aspect of the present invention, there are provided stabilized polypeptide particles comprising a pituitary adenylate cyclase polypeptide and a stabilizing agent selected from the group consisting of metal ions and sugars that are stable under acidic conditions at temperatures up to or exceeding physiological conditions, the polypeptide particles being formulated to exhibit an acidic reconstitution pH.

The solid-state polypeptide particles of the present invention include a polypeptide (a pituitary adenylate cyclase polypeptide) that is stable at acidic pH and are formulated to exhibit an acidic reconstitution pH. The pH of the aqueous stabilizing solution prior to particle formation (the "apparent pH") controls the pH exhibited by the subsequently formed particles (the "reconstitution pH") and defines the protonation state of the polypeptide within the solid-state polypeptide particles. For the purposes of the present invention, particles exhibiting an acidic reconstitution pH include polypeptide particles exhibiting a reconstitution pH below pH 5, with particles exhibiting a reconstitution pH below pH 4 being preferred and particles exhibiting a reconstitution pH ranging from pH 2 to pH 4 being particularly preferred. Controlling the pH exhibited by the solid-state polypeptide particles of the present invention allows control over the protonation state of the polypeptide included in the solid-state particles, and it has been found that formulating the solid-state polypeptide particles of the present invention at an acidic pH imparts a substantial stabilization effect. It is believed that formulating the solid-state polypeptide particles of the present invention at acidic pH stabilizes the polypeptide because the acidic environment favors protonation of amino groups included in the polypeptide. Often the amino groups included in a polypeptide are involved in the formation of reactive intermediates that are prone to inter- or intra-molecular reactions that alter the chemical nature of the peptide and function as a significant pathway of polypeptide degradation. By maintaining the polypeptide in an environment that favors protonation of the amino groups included in the polypeptide, it is believed that the polypeptide particles limit the involvement of amino groups in the formation of reactive intermediates and, as a result, limit the degradation of the polypeptide resulting from inter- or intra-molecular reactions.

The addition of metal ions to solid-state particles according to the invention provides additive polypeptide stabilization. Where the solid-state polypeptide particles according to the invention are formulated to include metal ions, the metal ions are preferably provided by a divalent metal salt, such as, CaCl₂, MgCl₂ or ZnCl₂. The precise amount of metal ion included in a solid-state polypeptide particle according to the invention will depend on the particular peptide to be stabilized, the pH of the particle, and the presence or absence of a stabilizing sugar. However, where a solid-state peptide particle according to the invention includes a metal ion, the particle is generally formulated such that the molar ratio of the metal ion to stabilized polypeptide is between about 1/1 and 10/1, with a molar ratio ranging from about 2/1 to about 6/1 being preferred, and a molar ratio of about 4/1 being particularly preferred. The addition of metal ions to the solid-state polypeptide particles of the present invention reduces the amount of polypeptide dimerization over time, and thereby imparts additive polypeptide stabilization. It is believed that the metal ions work to reduce polypeptide dimerization through the formation of ionic linkages or salt bridges with the polypeptide molecules, which ionic linkages or salt bridges limit the opportunity for interaction between polypeptide molecules.

The addition of a stabilizing sugar to solid-state peptide particles according to the invention also provides additive polypeptide stabilization. The inclusion of a stabilizing sugar imparts additive polypeptide stabilization by limiting polypeptide molecular mobility and reducing intermolecular interaction between polypeptide molecules. However, because the solid-state polypeptide particles of the present invention can be formulated to stabilize the polypeptide via two or more additive stabilizing conditions, where solid-state polypeptide particles according to the invention are formulated to include a stabilizing sugar, the amount of sugar required to achieve an acceptable level of stability is significantly reduced. In particular, where solid-state polypeptide particles according to the invention are formulated to include a stabilizing sugar, the sugar is generally included in an amount ranging from about 0. 1/1 wt/wt to about 1/1 wt/wt relative to the polypeptide stabilized. In preferred embodiments, solid-state polypeptide particles of the present invention including a stabilizing sugar will include the stabilizing sugar in amounts ranging from about 0.1/1 wt/wt to about 0.5/1 wt/wt or from about 0.1/1 wt/wt to about 0.25/1 wt/wt relative to the polypeptide stabilized. Where solid-state polypeptide particles according to the invention are formulated to include both a stabilizing sugar and metal ions, the amounts of sugar and metal ions required to achieve a desired degree of polypeptide stabilization may be reduced to relative formulations that include stabilizing sugar without metal ions or metal ions without stabilizing sugar. Such a potential advantage may be achieved due to the additive stabilizing effects of the sugar, the metal ions, and acidic environmental pH.

Though non-reducing sugars are generally useful as stabilizing agents in solid-state polypeptide particles according to the present invention, it has been found that not all sugars are suitable for stabilization of a peptide particle according to the invention. Specifically, it has been found that sucrose, a sugar commonly used in the stabilization of polypeptides, is not suitable for use under acidic conditions. In fact, when sucrose is used under such conditions, it has been found to produce a destabilizing effect. It is believed that such a destabilizing effect is generated because sucrose itself is not chemically stable in an acidic environment. In particular, sucrose undergoes hydrolysis into glucose and fructose at acidic pH. Although, trehalose is a disaccharide like sucrose, trehalose is chemically stable under acidic conditions and at elevated temperatures. Methyl-mannopyranoside ("methyl-MP"), a monosaccharide, is also chemically stable in an acidic environment. Moreover, both trehalose and methyl-MP have been found to provide significant additive polypeptide stabilization when included in solid-state polypeptide particles formulated to exhibit an acidic pH. Therefore, where solid-state polypeptide particles according to the invention are formulated to include a stabilizing sugar, the sugar included in the formulation must be stable under acidic conditions and is preferably stable under acidic conditions at temperatures up to and exceeding physiological conditions.

Solid-state polypeptide particles according to the invention can be prepared from an aqueous stabilizing solution. To prepare an aqueous stabilizing solution suitable for preparing solid-state polypeptide particles according to the invention, the polypeptide to be stabilized is dissolved in an acidic solution. An acidic aqueous stabilizing solution may be achieved through the addition of a suitable acid, such as HCl, in an amount that provides a solution having a desired pH. The pH of an aqueous stabilizing solution plays a crucial role in stabilizing the polypeptide contained within the solid-state polypeptide particles that are produced using the aqueous stabilizing solution. In order to achieve solid-state polypeptide particles according to the invention (i.e., having an acidic reconstitution pH), it may be necessary to prepare the aqueous stabilizing solution at an apparent pH that is significantly lower than the targeted reconstitution pH. An aqueous stabilizing solution suitable for preparing solid-state polypeptide particles includes a metal salt or stabilizing sugar dissolved therein. The amount of metal salt or stabilizing sugar dissolved in an aqueous stabilizing solution suitable for producing solid-state polypeptide particles according to the invention may vary. However, where the aqueous stabilizing solution is to be used to prepare solid-state polypeptide particles including a stabilizing sugar, the aqueous stabilizing solution will preferably include the desired stabilizing sugar at an amount ranging from about 0.1/1 wt/wt to about 1/1 wt/wt relative to the polypeptide to be stabilized. Moreover, where the aqueous stabilizing solution is to be used to prepare solid-state polypeptide particles including a metal ion, the aqueous stabilizing solution is preferably prepared to include the desired metal ion at a molar ratio of metal ion to polypeptide ranging from about 1/1 to 10/1.

In order to form solid-state polypeptide particles according to the invention from an acidic aqueous stabilizing solution, the stabilizing solution is subjected to a known particle formation process. For example, an aqueous stabilizing solution may be processed using a lyophilization or spray drying process to achieve solid-state peptide particles. In a one example, solid-state polypeptide particles are prepared from an aqueous stabilizing solution using a lyophilization cycle that includes freezing the stabilizing solution at 4° C for 30 minutes and at -50° C for 3 hours (with a cooling rate of 2.5°C/min). After freezing the aqueous stabilizing solution as described, a primary drying cycle is conducted at a chamber pressure of 50mT at -30°C for 10 minutes and then at 0°C for 10 hours. The primary drying cycle is followed by a secondary drying cycle at a chamber pressure of 200 mT at 0°C for 3 hours and then at 20°C for 12 hours and at 30°C for 6 hours, with all temperature levels ramped at 0.5°C/min. However, the present invention is not limited to solid-state polypeptide particles produced by the precise lyophilization process described herein. Several suitable lyophilization and spray drying processes are known in the art of protein particle formation and may be applied to provide solid-state polypeptide particles according to the present invention.

Also described in this application are solid-state polypeptide particles formulated to exhibit a near neutral reconstitution pH. As it is used herein, the term "near neutral" refers to a pH ranging from pH 5 to about pH 8. Preferably, solid-state polypeptide particles according to this second embodiment (which falls outside the scope of the present invention) are formulated to exhibit a reconstitution pH of between about pH 5 and pH 7. Polypeptide particles according to the second embodiment include a buffer and further include a surfactant, a stabilizing sugar, a metal ion, or both a stabilizing sugar and a metal ion. It has been found that formulating solid-state polypeptide particles to exhibit near neutral reconstitution pH and to include buffering excipients significantly stabilizes the polypeptide material included in the solid-state particles. It is believed that at near neutral pH a buffer may serve to stabilize the polypeptide included in solid-state particles through a counter ion effect. In particular, it is thought that the buffer material may interact with or bind to one or more sites included in the polypeptide that exhibit a tendency to react inter- or intra-molecularly in such a way that such sites are no longer available to react.

Buffers suitable for use in formulating solid-state polypeptide particles according to the second embodiment (which falls outside the scope of the present invention) are those that buffer at near neutral pH. Specific examples of buffers that may be used in solid-state polypeptide particles according to the second embodiment include amino acid or peptide buffers, such as histidine buffer (His-6) or histidine-glutamic acid (His-Glu) buffer, and inorganic buffers, such as succinate and citrate buffers. Solid-state polypeptide particles according to the second embodiment are generally formulated with up to about 20% buffer by weight, with polypeptide particles including up to about 15% buffer by weight being preferred. However, the precise amount of buffer included in solid-state polypeptide particles according to the second embodiment may vary according to the amount and type of polypeptide included in the particles. Moreover, the amount and type of buffer used to formulate solid-state polypeptide particles according to the second embodiment may also be adjusted order to achieve solid-state polypeptide particles exhibiting a desired reconstitution pH.

By formulating solid-state particles according to the second embodiment (which falls outside the scope of the present invention) to include a stabilizing sugar, a metal ion, or both a stabilizing sugar and a metal ion, the stabilization of the polypeptide included in the particles increases beyond that achieved by simply formulating the polypeptide in a near neutral environment in the presence of a buffer. Where solid-state polypeptide particles are formulated according to the second embodiment and include a stabilizing sugar, a metal ion, or both a stabilizing sugar and a metal ion, the amount of sugar and metal ion included in the particles preferably falls within the ranges detailed in relation to the solid-state peptide particles of the invention. In particular, if solid-state polypeptide particles according to the second embodiment are formulated to include a stabilizing sugar, the amount of stabilizing sugar included in the particles preferably ranges from about 0.1/1 wt/wt to about 1/1 wt/wt relative to the polypeptide to be stabilized. Again, non-reducing sugars are generally useful as stabilizing sugars in solid-state polypeptide particles according to this second embodiment (which falls outside the scope of the present invention), but because they are formulated at near neutral pH, however, solid-state polypeptide particles according to the second embodiment may incorporate stabilizing sugars, such as sucrose, that are not stable in an acidic environment. Further, if solid-state polypeptide particles according to the second embodiment are formulated to include a metal ion, the particles are preferably formulated to include the desired metal ion at a molar ratio of metal ion to polypeptide ranging from about 1/1 to about 10/1. Where solid-state polypeptide particles according to the second embodiment are formulated to include both a stabilizing sugar and metal ions, the amounts of sugar and metal ions required to achieve a desired degree of polypeptide stabilization may be reduced relative to formulations that include stabilizing sugar without metal ions or metal ions without stabilizing sugar. Such a potential advantage may be achieved due to the additive stabilizing effects of the sugar, the metal ions, and buffer conditions at near neutral pH.

Where solid-state peptide particles according to the second embodiment (which falls outside the scope of the present invention) are formulated to include a surfactant, the surfactant used is preferably an anionic surfactant, such as sodium dedocyl sulfate (SDS). The amount of surfactant included in solid-state polypeptide particles according to the second embodiment may vary according to the amount and type of polypeptide to be stabilized as well as the nature and amount of other excipients included in the particles. Nevertheless, where solid-state polypeptide particles according to the second embodiment are formulated from an aqueous stabilizing solution containing the desired surfactant in an amount ranging from about 0.02 wt% up to about 0.2 wt%.

Solid-state polypeptide particles according to the second embodiment (which falls outside the scope of the present invention) can be prepared from an aqueous stabilizing solution. To prepare an aqueous stabilizing solution suitable for preparing solid-state polypeptide particles according to the second embodiment, the polypeptide to be stabilized is dissolved in a solution exhibiting a near neutral apparent pH and including a surfactant, a metal salt, a stabilizing sugar, or both a metal salt and a stabilizing sugar, as desired. A near neutral stabilizing solution may be achieved through the addition of a suitable buffer, such as those buffers already discussed, in an amount that provides a solution having a desired apparent pH. As is true of aqueous stabilizing solutions used to prepare solid-state polypeptide particles according to the invention, the apparent pH of an aqueous stabilizing solution used to create solid-state polypeptide particles according to the second embodiment (which falls outside the scope of the present invention) controls the reconstitution pH of the solid-state polypeptide particles produced using the aqueous stabilizing solution. Therefore, the amount and type of buffer used in an aqueous stabilizing solution for producing solid-state polypeptide particles according to the second embodiment may be varied in order to achieve a desired apparent pH and a desired reconstitution pH. Moreover, in order to achieve solid-state polypeptide particles that are characterized by a buffer content within the ranges described herein, the amount of buffer included in the aqueous stabilizing solution will vary depending upon, among other factors, the type of buffer used, the precise amount of buffer desired in the solid-state particles, and the nature and amounts of the polypeptide and other excipients to be included in the solid-state particles. For example, in the Examples that are detailed below, stabilizing solutions characterized by a 10mM concentration of histidine buffer provided solid-state polypeptide particles containing 14 wt% buffer material.

The amount of metal salt or stabilizing sugar dissolved in an aqueous stabilizing solution suitable for producing solid-state polypeptide particles according to the second embodiment (which falls outside the scope of the present invention) may also vary depending on the amount and nature of the polypeptide to be stabilized as well as the nature of the metal salt or stabilizing sugar. However, where an aqueous stabilizing solution for preparation of solid-state polypeptide particles according to the second embodiment includes a stabilizing sugar, the aqueous stabilizing solution will preferably include the desired stabilizing sugar at an amount falling within the ranges already described (i.e., from about 0.1/1 wt/wt to about 1/1 wt/wt relative to the polypeptide to be stabilized, with the ranges of from about 0.1/1 wt/wt to about 0.5/1 wt/wt and from about 0.1/ 1 wt/wt and 0.25/1 wt/wt being preferred). Moreover, where an aqueous stabilizing solution for preparation of solid-state polypeptide particles according to the second embodiment includes a metal ion, the aqueous stabilizing solution is preferably prepared to include the desired metal ion at a molar ratio of metal ion to polypeptide falling within the ranges already described (i.e., a molar ratio of metal ion to polypeptide ranging from about 1/1 to about 10/1, with the range of about 2/1 to about 6/1 being preferred and a molar ratio of about 4/1 being particularly preferred).

In order to form solid-state polypeptide particles according to the second embodiment (which falls outside the scope of the present invention) from a near neutral aqueous stabilizing solution, the near neutral stabilizing solution is subjected to a known particle formation process. As described in relation to the formation of particles according to the invention, solid particles according to the second embodiment may be formed from an appropriately formulated aqueous stabilizing solution using a lyophilization or spray drying process. For example, the lyophilization process described herein is suitable for providing solid-state polypeptide particles according to the second embodiment from an aqueous stabilizing solution. Again, however, the disclosure is not limited to the use of the particular lyophilization process detailed. Particle formation through lyophilization and spray drying processes is well known in the art and any suitable lyophilization or spray drying process may be applied to the aqueous stabilizing solutions of the second embodiment (which fall outside the scope of the present invention) to provide solid-state polypeptide particles.

The solid-state polypeptide particles, the aqueous stabilizing solutions, and the methods disclosed herein are particularly well suited to the stabilization of peptides belonging to the superfamily of peptides including Pituitary Adenylate Cyclase Polypeptide ("PACAP") and glucagon. In humans, the PACAP/glucagon superfamily of peptides includes at least nine different types of bioactive peptides: PACAP-27; PACAP-38; glucagon; glucagon-like peptides, such as GLP-1 and GLP-2; growth hormone releasing factor ("GRF"); vasoactive intestinal polypeptide ("VIP"); peptide histidine methionine ("PHM"); secretin; and glucose-dependent insulinotropic polypeptide ("GIP"). Eight of these nine different types of bioactive peptides are found in the brain and are classified as neuropeptides. In addition many members of the PACAP/glucagon superfamily are present in the gastrointestinal, pancreatic, and gonadal organs (*See*, The origin and function of the pituitary adenylate cyclase-activating polypeptide (PACAP)/glucagon superfamily, Endocrine Reviews, 21(6):619-670). The peptides included in the PACAP/glucagon superfamily are related in structure by their N-terminal amino acids, and the genes and precursor molecules used in the cellular production of the various different peptides included in the PACAP/glucagon superfamily are similar in structure.

Of particular interest is a synthetic PACAP-R3 agonist analog ("the PACAP analog") manufactured by Bayer Corporation ("Bayer"). This synthetic peptide is defined by the following 31 amino acid sequence: HSDAVFTDNY TRLRKQVAAK KYLQSIKNKR Y. Bayer engineered the PACAP analog for potency and selectivity to the R3 receptor in the pancreas. To improve stability of the PACAP analog, the original methionine at position 17 was replaced with a valine and the asparagine originally located at position 24 was replaced with a glutamine. The PACAP analog is useful in treating Type II Diabetes, and it would be desirable to deliver the PACAP analog from an implantable drug delivery system capable of delivering the PACAP analog to a subject (humans) at therapeutic doses over a period of at least three months, and preferably at least 6 months. However, whether it is maintained in a solid state or dissolved in aqueous or organic solvent solutions, unprotected PACAP analog is unstable at temperatures that approach or exceed physiological conditions. Therefore, in order to load the PACAP analog in a suspension suitable for delivery from an implantable, extended release delivery systems, the solid-state PACAP analog particles must be stabilized against the degradation that would otherwise result from extended exposure to temperatures that approximate or exceed physiological conditions.

As solid-state PACAP analog is exposed to temperatures approximating or exceeding physiological conditions, the most significant source of degradation has been determined to be aggregate formation. In particular, using lyophilized PACAP analog, it was determined that the major degradation products for unprotected, lyophilized PACAP analog are covalent dimers, dimer-OAc and PACAP-OAc adducts. Presently it is believed that the degradation of PACAP analog into such aggregate products proceeds via reactive intra-molecular cyclic imide formation followed by nucleophilic attack by acetate ions, by another molecule of PACAP analog, or by both. Likely sites of modification for these proposed degradation paths are at the Asp3Ala4 and the Gln24Ser25 amino acids of the PACAP analog molecule. It has been further determined that N-terminal peptide bond cleavage also occurs to degrade solid-state, unprotected PACAP analog. This proteolysis was found predominantly at pH4 and presumably occurs via assisted intra-molecular ring closure of the Asp3 carboxyl side chain. However, as is detailed in some of the examples that follow, embodiments of the solid-state polypeptide particles of the present invention are effective in stabilizing PACAP analog against degradation, even when the particles are maintained at temperatures of 40°C and 60° C over a period of months.

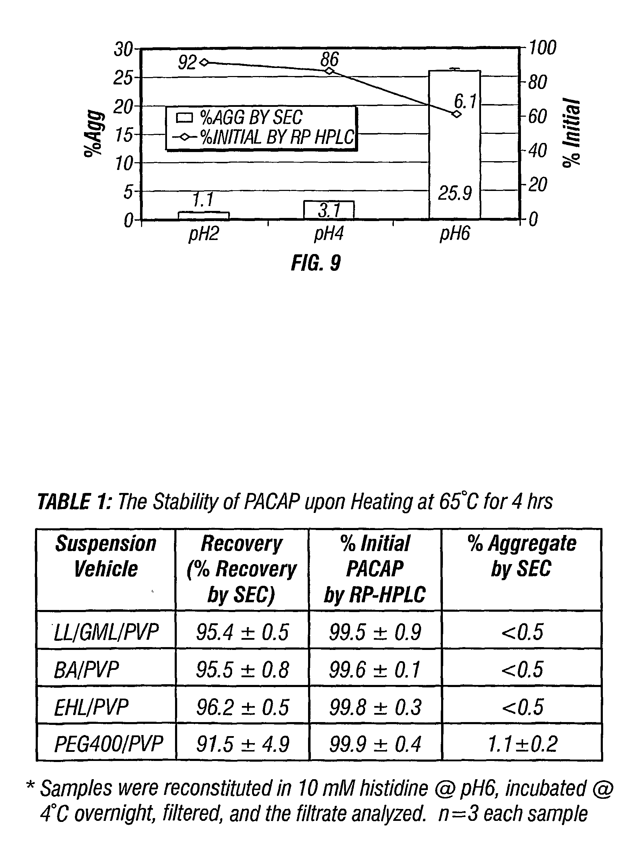

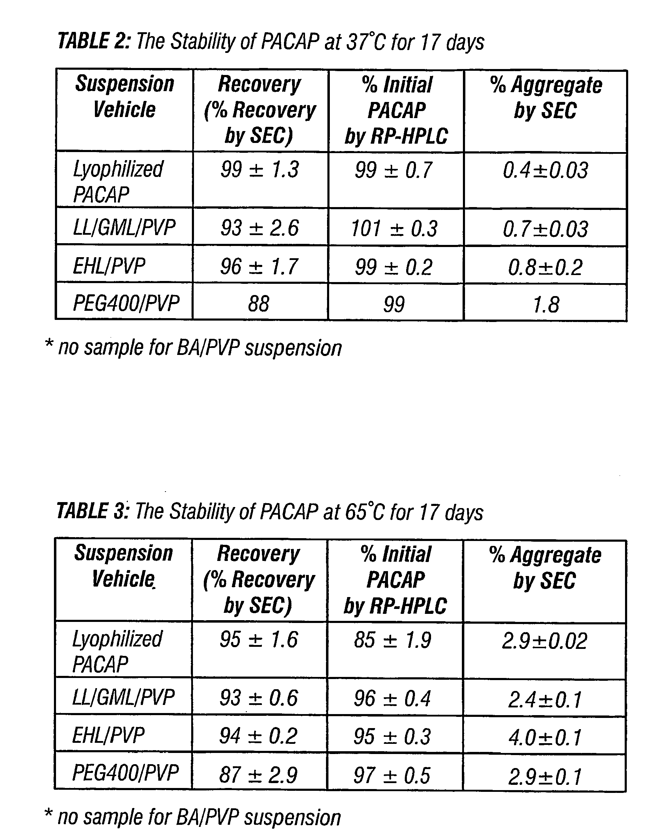

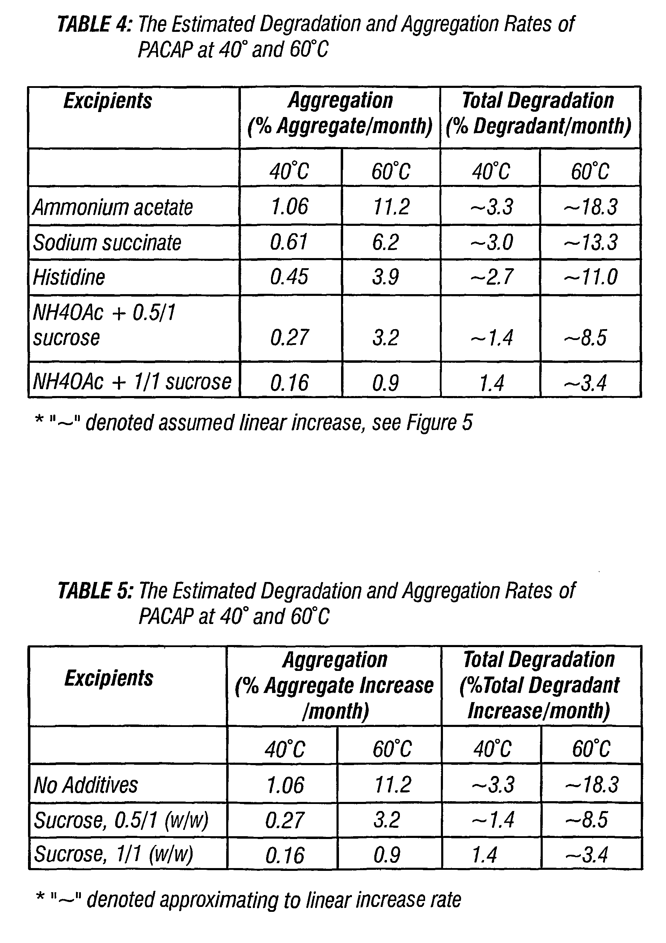

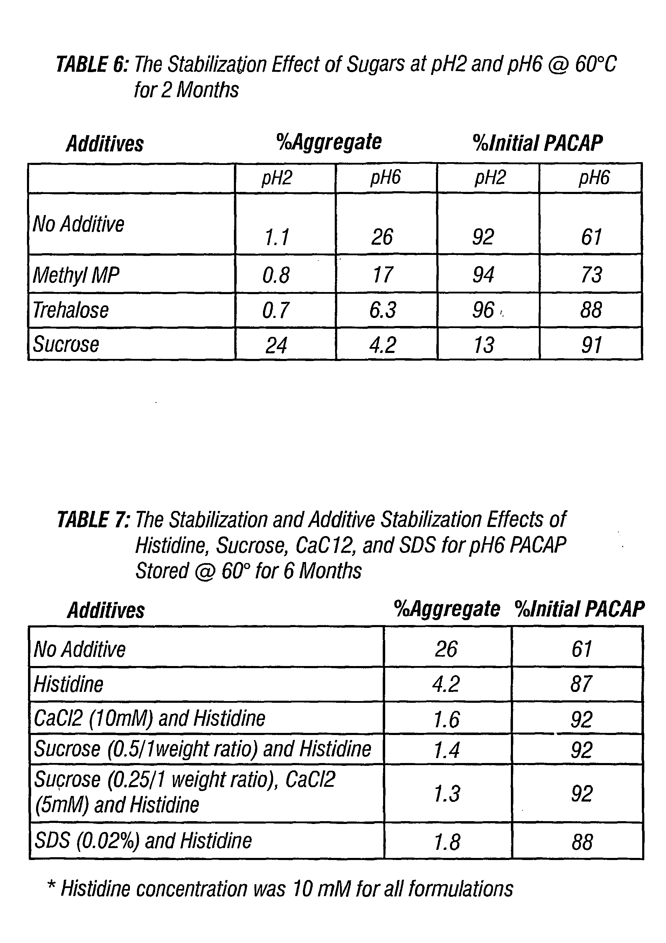

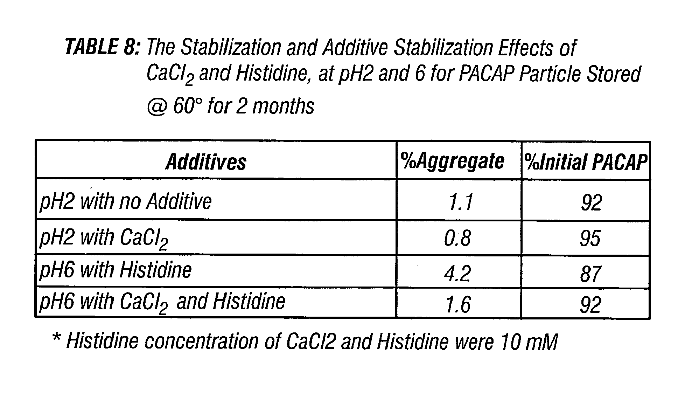

Though several different examples of the method of preparation of solid-state polypeptide particles provide effective stabilization of PACAP analog, three particular examples provide superior stabilization. In one example, solid-state PACAP analog particles are lyophilized from a stabilizing solution exhibiting an apparent pH of 2. The solvent for the stabilizing solution is formed using H₂O having the pH adjusted to the desired value using diluted HCL. The solid-state PACAP analog particles according to this example allow 92% recovery of the initial PACAP analog and permit only 1.1% dimer formation after two months of storage at 60° C. Figure 9 presents the two-month stability results, which demonstrate the greatly improved stability of solid-state PACAP particles prepared from a stabilizing solution having an apparent pH of 2.

In a second example, the solid-state polypeptide particles are formulated at acidic pH and include trehalose and PACAP analog at a 0.55/1 w/w ratio (trehalose/PACAP analog). These particles allow 96% recovery of the initial PACAP analog and allow only 0.7% dimer formation, even after two months of storage at 60° C. To formulate solid-state PACAP analog particles characterized by an acidic pH and including trehalose and PACAP analog at a 0.55/1 w/w ratio, an acidic aqueous stabilizing solution may be used. Such a stabilizing solution can be prepared using a suitable acid, such as diluted HCl. The pH of such a solution (the apparent pH) is preferably adjusted to a pH of about 2 and the trehalose and PACAP analog are dissolved in the solution at the desired 0.55/1 w/w ratio. The prepared solution may then subjected to a lyophilization or spray drying process to provide the desired solid-state PACAP analog particles.

In a third example, the solid state polypeptide particles are again formulated at acidic pH and include PACAP analog and Ca²⁺ ions at a 4/1 molar ratio (Ca²⁺/PACAP analog). After two months of storage at 60° C, particles formed according to this formulation provide 95% recovery of the initial PACAP analog and allow only 0.8% dimer formation. To formulate solid-state PACAP analog particles characterized by an acidic pH and including Ca²⁺ and PACAP analog at a 4/1 molar ratio, an acidic aqueous stabilizing solution may be prepared. Again, such a solution may be prepared using a suitable acid, such as diluted HCl, and the pH of the solution is preferably adjusted to a of about pH 2. The appropriate amount of Ca²⁺ ions and PACAP analog may be provided in solution by dissolving amounts of CaCl2 and PACAP that result in a solution having a molar ratio of Ca²⁺ to PACAP analog of 4/1. The prepared stabilizing solution may then be subjected to a lyophilization or spray drying process to provide the desired solid-state PACAP analog particles.

Though the stability of the PACAP analog contained within the particles described herein has been evaluated for a maximum period of two months at a maximum temperature of 60° C, the findings provided by such evaluations and a comparison of degradation kinetics at 40° C indicate that the method is suitable for providing solid-state PACAP analog particles that are stabilized against degradation well beyond two months at temperatures that approximate physiologic temperature *(i.e.,* 40° C). Specifically, it has been found that the total rate of degradation of PACAP analog contained within particles is approximately five times higher at 60° C than at 40° C. Moreover, the rate of aggregation or dimerization within the solid-state PACAP particles produced is ten times higher at 60° C than at 40°C. Therefore, where solid-state PACAP analog particles produced by the method disclosed herein are maintained at approximately 37° C, it is anticipated that the PACAP analog contained within the particles will demonstrate acceptable stability for up to six months and likely longer.

For instance, the method disclosed herein may be used to provide stabilized, solid-state particles of other members of the PACAP/glucagon superfamily, particularly those family members, such as human VIP and human growth hormone releasing factor, which that have substantially homologous amino acid sequences. However, the method is also not limited to stabilization of members of the PACAP/glucagon superfamily. The solid-state polypeptide particles, aqueous stabilizing solutions, and methods disclosed herein may be useful for stabilizing any polypeptide that exhibits degradation due to proteolysis or aggregate formation or has primary structures prone to degradation pathways, such as those observed for PACAP.

### EXAMPLE 1 (COMPARATIVE)

Due to the nature of the suspension vehicles used in implantable drug delivery systems, mixing of the suspension formulation bulk and subsequent loading of the suspension formulation into the implantable systems are often conducted at elevated temperatures. To evaluate the stability of PACAP analog (or simply "PACAP") suspended in various suspension vehicles at elevated temperatures, unprotected, lyophilized PACAP was suspended in four different suspension vehicles (LL/GML/PVP, BA/PVP, EHL/PVP, and PEG/PVP). To suspend the PACAP into the suspension vehicles 3.3 mg PACAP acetate was manually mixed into each of the different suspensions in achieve a PACAP content of approximately 3%. The stability of the PACAP suspension formulations was then evaluated after incubation at 65° C for four hours. The stability of the prepared PACAP suspensions was evaluated by RP-HPLC and SEC, and as can be seen by reference to table 1, the results showed that PACAP was stable throughout the four hour incubation period and, therefore, should survive the temperature conditions typically associated with the suspension formulation manufacturing processes.

The PACAP suspensions were then further incubated at 37° C and 65° C an additional 17 days. After the additional 17-day incubation period, a marginal stability loss was observed in the suspensions incubated at 37°C (Table 2), while a relatively more aggressive degradation was observed in the suspensions incubated at 65°C (Table 3). The results revealed covalently linked aggregation as the major degradation pathway in the evaluated PACAP suspensions. The fact that aggregation occurred with and without the presence of vehicle suggests that the suspension vehicle was not responsible for the resultant self-association. In addition other chemical degradation contributed to the loss of lyophilized PACAP.

### EXAMPLE 2 (COMPARATIVE)

The preliminary findings from the processing study led to the evaluation of the stabilization effect of sugar (sucrose) and non-ionic surfactant (Tween 80) on PACAP lyophiles and suspensions. In order to carry out the evaluation, samples were prepared with PACAP in 10 mM NH₄OAc (pH6.4) at 3 levels of Tween 80 (0, 0.05, 0.2wt%) and sucrose (0, 0.5/1, 1/1, w/w). A solution of PACAP in 10 mM of histidine (pH 6.4) and sodium succinate (pH 5.6) was also prepared. Each of the PACAP solutions were lyophilized to obtain reconstituted, solid-state PACAP particles, and individual vials were provided 3.3 mg samples of the material reconstituted from each of the solutions, with the samples containing (1) no additive, (2) 0.2wt% Tween 80, (3) 0.2%wt Tween 80+0.5:1, w/w sucrose, and (4) 1:1, w/w sucrose. The various 3.3 mg samples were manually mixed with LL/GML/PVP and BA/PVP to provide suspension formulations having about 3% PACAP content, and the suspension formulations were subjected to 4 hours of incubation at 65°C. After the initial incubation period, the various suspension formulations were stored as described below and evaluated by RP-HPLC and SEC.

| **Storage Temp/Time** | **0** | **24 days** | **3 month** |
|---|---|---|---|
| 2-8°C | x | - | x |
| 40°C | - | x | x |
| 60°C | - | x | x |

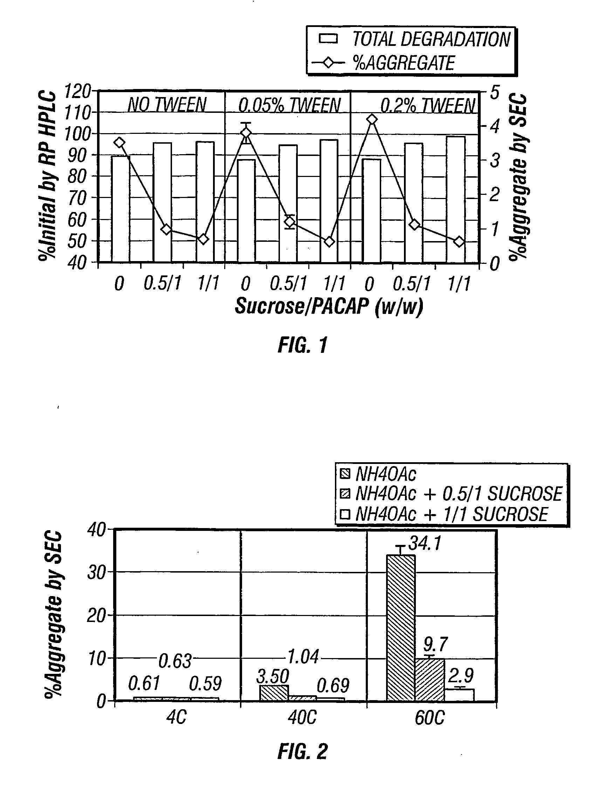

Figure 1 shows that sucrose suppressed PACAP aggregation while the presence of Tween 80 had no added effect. The overall stability, assessed by percent of PACAP remaining, also increased accordingly. The extent of PACAP aggregation was shown to be a function of storage temperature (Figure 2). Lyophilized PACAP without a stabilizer was stable for at least 3 months at 4°C and aggregated substantially more rapidly at 60°C compared to 40°C. This study demonstrated that sucrose stabilized lyophilized PACAP both at the physiological (40°C) and accelerated (60°C) temperatures.

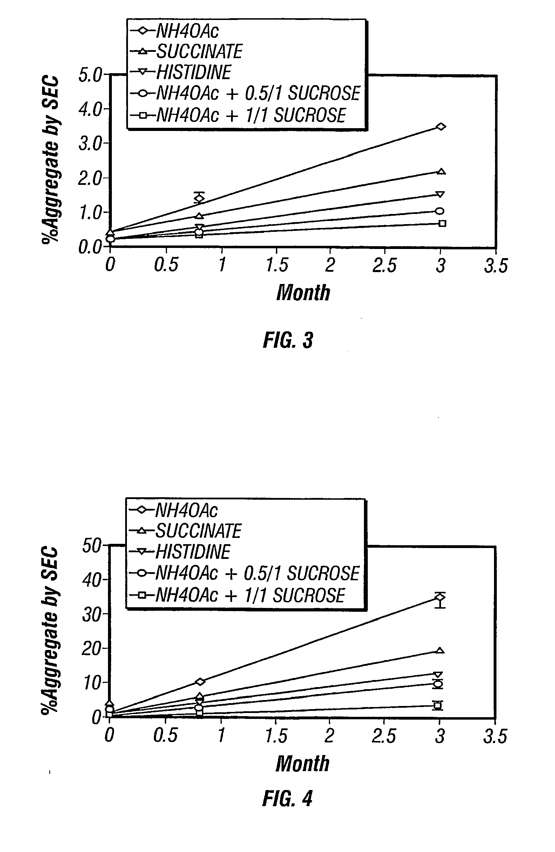

Figures 3 and 4 show a linear increase of PACAP aggregate formation over 3 months at 40 and 60°C, respectively, for the lyophilized formulations prepared with various different excipients. Histidine and sodium succinate proved to be better stabilizers than ammonium acetate. Except in the samples containing 1:1 w/w sucrose, the rate of aggregation at 60° C was approximately 10 times faster than that observed at 40° C, and the rate of total degradation was about 5 times faster at 60°C than that observed at 40°C (Table 4). These results indicate that high sucrose content increased thermal stability by decreasing overall molecular mobility and protein-protein interaction.

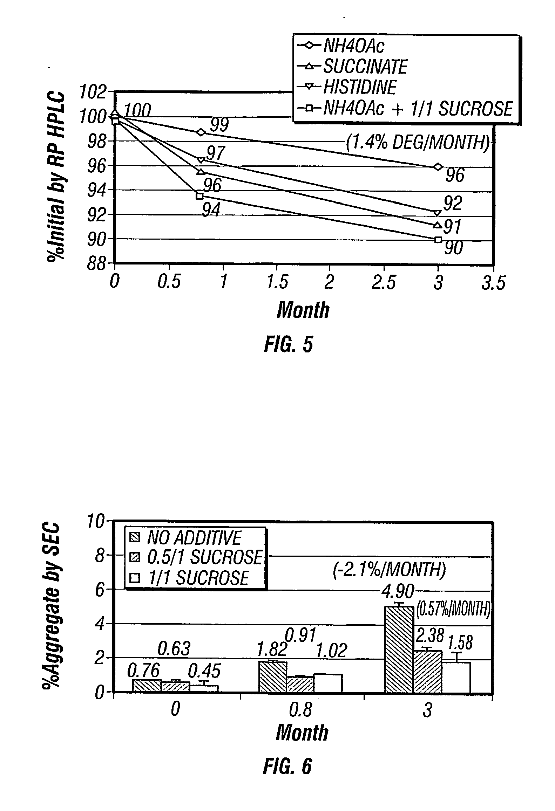

The total degradation, on the other hand, followed a less linear relationship over a 3-month period of time (Figure 5). This experiment demonstrated that lyophilized PACAP (prepared at an apparent pH of 6.4 in ammonium acetate) containing an equal amount of sucrose (w/w) resulted in the best (but not acceptable) stability, with an estimated 8.4% the total degradation in 6 months at 40°C.

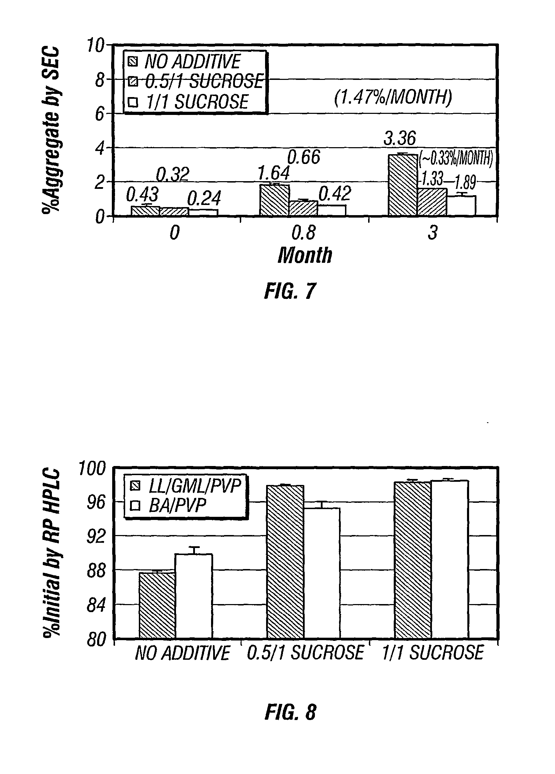

In addition, this experiment showed that sucrose suppressed aggregate formation for PACAP suspensions in BA/PVP and LL/GML/PVP (Figures 6 and 7) similarly to lyophilized PACAP formulations alone. The stabilization effect of sucrose in respect to aggregate formation appeared to be reduced in the BA/PVP suspension. Also a higher amount of sucrose was needed for a similar longer-term stability of PACAP in BA/PVP (Figure 8).

In order to characterize the degradation products and elucidate the degradation paths, the reconstituted stability sample (lyophilized PACAP stored at 60°C for 24 days) was analyzed by electrospray-time-of-flight mass spectrometry and the degradation products were identified using an RP-HPLC process.

A molecular weight (MW) of 3743 amu was assigned to the intact PACAP. Based on the RP-HPLC analysis, the major degradation products were identified to be covalent dimer and dimer-OAc adducts. Significantly, a cluster of peaks, which elute after the main peak but prior to the elution of the dimer peaks, all have a mass that is equal to that for PACAP-OAc adduct (*i.e.,* 3785 amu,). The combined results from the RP-HPLC and the electrospray-time-of-flight mass spectrometry -analyses suggest that both degradations were rendered through the same reactive intermediate(s). Based on this analysis, the potential degradation mechanism can be postulated as follows:

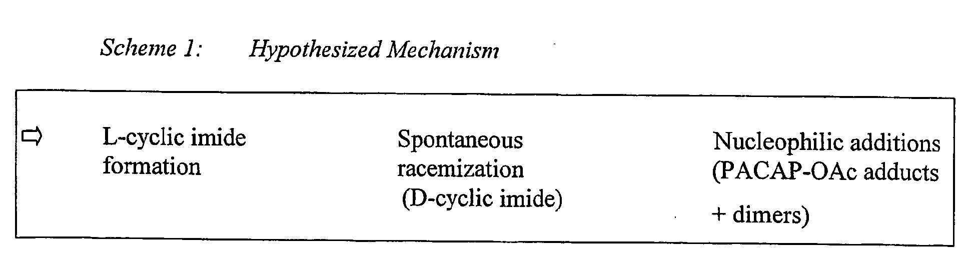

It can be hypothesized that the degradation proceeds by a two-step mechanism via the rate-limiting cyclic imide intermediate formation followed by a nucleophilic addition of an acetate ion, another PACAP molecule, or both. Since the water content was low for all samples (<1 %), deamidation as a result of nucleophilic addition of water was not predominant

### EXAMPLES 3 (COMPARATIVE)

Solid-state PACAP particles were prepared via a lyophilization process (FTS Duro stop) from three different polypeptide solutions prepared in NH4OAc buffer at pH6. Each of the three different solutions contained a different amount of sucrose, with the first containing no sucrose, the second containing a weight ratio of sucrose to PACAP of 0. 5/1, and the third containing a weight ratio of sucrose to PACAP 1/1. Solid-state PACAP particles prepared from each of the three solutions were stored at control temperature ranging from 2° C to 8° C, a temperature approximating physiologic temperature (40° C), and a temperature exceeding physiologic temperature (60° C). The stability of the PACAP contained within each group of solid-state PACAP particles was assessed at 24 days and 3 months. To assess PACAP stability within each group of particles, the particles were reconstituted in water and analyzed by reversed phase RP-HPLC (acetonitrile gradient elution with mobile phase containing 0.1% trifluoroacetic acid, and a UV detection at 214 nm) and size exclusion HPLC (SEC, isocratic elution, 30% acetonitrile mixed with 70% of aqueous solution containing 0.1 % trifluoroacetic acid and 200 mM of sodium chloride, and a LIV detection at 220 nm).

The rate of aggregation (assessed by SEC) was found to be 10 times higher at 60° C than at 40° C for all formulations tested (Table 5). Except in solid-state PACAP particles prepared with a PAGAP to sucrose weight ratio of 1/1, the rate of total degradation (assessed by RP HPLC) observed in the various solid state PACAP particles was about 5 times faster at 60° C that at 40° C.

### EXAMPLE 4 (COMPARATIVE)

The effect of pH on the stability of PACAP molecules over time was evaluated. In order to make such an evaluation, PACAP raw material was first dissolved in H₂O in three different solutions, with the first solution having a pH of 2, the second solution having a pH of 4, and the third solution having a pH of 6. The pH of each solution was adjusted to the desired value using diluted HCL. Each of the three solutions was placed in lyophilization vials, and solid-state PACAP particles were produced from each of the three solutions through the lyophilization process already described herein. Particles from each of the three solutions were then stored for two months at 60° C, and the stability of the PACAP included in the solid-state particles was thereafter evaluated using RP-HPLC and SEC. Figure 9 presents the two-month stability results, which demonstrate the greatly improved stability of solid-state PACAP particles prepared at acidic pH.

### EXAMPLES 5

The stability of PACAP included in solid-state PACAP particles prepared at varying pH with an amount of potentially stabilizing sugar was evaluated. In each instance the solid-state PACAP particles consisted of a 7.3 molar equivalent of one of three different sugars: sucrose; trehalose; and methyl mannopyranoside (all from Sigma). The solid-state PACAP particles evaluated were prepared from eight different aqueous solutions. The first two (comparative) aqueous solutions were prepared by dissolving PACAP into a first solution having a pH adjusted to 2 and a second solution having a pH adjusted to 6 (as before the pH of the various solutions described in this Example was adjusted using diluted HCl). The third aqueous solution was prepared by dissolving both PACAP and methyl-MP into a solution having a pH adjusted to 2 and the fourth (comparative) aqueous solution was prepared by dissolving both PACAP and methyl-MP into a solution having a pH adjusted to 6. The fifth aqueous solution was prepared by dissolving both PACAP and trehalose into a solution having a pH adjusted to 2 and the sixth (comparative) aqueous solution was prepared by dissolving both PACAP and trehalose into a solution having a pH adjusted to 6. And the seventh and eighth (comparative)aqueous solutions were prepared by dissolving both PACAP and sucrose into a solution having a pH adjusted to 2 and into a solution having a pH adjusted to 6.

solid-state PACAP particles were prepared from each of the eight solutions through lyophilization, as has already been described, and solid-state PACAP particles produced from each of the eight solutions were then stored at 60° C for two months. After exposing the various solid-state PACAP particles to 60° C over two months, the stability of the PACAP included in various particles was evaluated using RP HPLC and SEC processes.

The HPLC analyses indicated all three sugars investigated showed improved stability when included in PACAP particles prepared at pH 6. In particular, at pH 6, sucrose provided roughly a 6-fold improvement in preventing aggregate formation and trehalose provided roughly a 4-fold improvement in the same measure. Moreover, both trehalose and methyl MP provided additive stability to the PACAP included in particles prepared at pH2 (Table 6) relative to PACAP particles prepared at pH 2 without the addition of a stabilizing sugar. However, when included in PACAP particles prepared at pH 2, sucrose exhibited a significant destabilizing effect. The destabilization effect of sucrose for PACAP prepared at pH2 was believed to be a result of sucrose decomposition during the process of producing the PACAP particles from a solution having an acidic pH.

### EXAMPLE 6 (COMPARATIVE)

PACAP particles containing histidine buffer (Sigma) and PACAP particles containing histidine buffer in combination with calcium chloride (CaCl₂, JT Baker), sucrose and sodium dedocyl sulfate (SDS, Pierce) were prepared and the stabilization of PACAP provided by such particles was evaluated. To prepare the PACAP particles evaluated, six different aqueous solutions were prepared. Each solution included a desired amount of FACAP dissolved therein, and the pH of each of the six solutions prepared was adjusted to 6 by the addition of diluted HCl. The first aqueous was prepared without additives (histidine buffer, CaCl₂, sucrose, or SDS). The second aqueous solution was prepared with a 10 mM concentration of histidine buffer. The third aqueous solution was formulated with a 10 mM concentration of histidine buffer as well as a 10 mM concentration of CaCl₂. The fourth aqueous solution was formulated with a 10 mM concentration of histidine buffer and included sucrose at a 0.5/1 weight ratio (sucrose/PACAP) relative to PACAP. The fifth aqueous solution was prepared with a 10 mM concentration of histidine buffer, a 10 mM concentration of CaCl₂, and an amount of sucrose providing a 0.5/1 weight ratio (sucrose/PACAP) relative to PACAP. The sixth aqueous solution was formulated with a 10 mM concentration of histidine buffer and 0.02 wt% SDS. Solid-state PACAP particles were then produced from each of the six solutions using the lyophilization process already described herein. The PACAP particles produced from each solution were then stored at 60 °C for two months, and the stability of the PACAP contained within the various different particles was evaluated using RP-HPLC and SEC processes after such storage. As can be seen in Table 7, the presence of histidine buffer greatly suppresses PACAP aggregate formation (a roughly 6-fold decrease in aggregate formation). In addition, sucrose, CaCl₂ and SDS further stabilized the peptide, providing roughly 14 fold to 20 fold decreases in aggregate formation.

### EXAMPLE 7

To evaluate the stabilizing effect of CaCl₂ in PACAP particles prepared at acidic and near neutral pH, four different types of particles were formulated, stored at 60 °C for two months, and then analyzed for PACAP degradation. The four different particle formulations were prepared from four different aqueous solutions. Each of the four solutions included a desired amount of PACAP dissolved therein and the pH of each solution was adjusted to the desired level using diluted HCI. The first aqueous solution was formulated without additives (histidine or CaCl₂), and the pH of the first solution was adjusted to 2. The second aqueous solution was formulated with a 10 mM concentration of CaCl₂, and the pH of the second solution was adjusted to 2. The third (comparative) aqueous solution was formulated with a 10 mM concentration of histidine buffer, and the fourth (comparative) aqueous solution was formulated with a 10 mM concentration of histidine buffer as well as a 10 mM concentration of CaCl₂. Solid-state PACAP particles were prepared from each of the four aqueous solutions (the composition of each of the different particles is outlined in Table 8) using the lyophilization process already described, and PACAP particles produced from each of the four solutions were stored at 60 °C for two months. After storage of the particles at 60 °C for two months, the stability of PACAP included in particles was evaluated using RP-HPLC and SEC processes. As can be seen in Table 8, the results of such evaluation demonstrated that CaCl₂ further improved the stability of PACAP particles at both pH 2 and 6 (Table 8).

## Claims

1. Stabilized polypeptide particles comprising a pituitary adenylate cyclase polypeptide and a stabilizing agent selected from the group consisting of metal ions and sugars that are stable under acidic conditions at temperatures up to or exceeding physiological conditions, the polypeptide particles being formulated to exhibit an acidic reconstitution pH.

2. The polypeptide particles of claim 1, wherein the polypeptide particles comprise a pituitary adenylate cyclase polypeptide, a metal ion and a sugar that is stable at acidic pH at temperatures up to and exceeding physiological conditions.

3. The polypeptide particles of claim 1 or 2, wherein the stabilizing agent is selected from disaccharides and monosaccharides that are stable under acidic conditions at temperatures up to and exceeding physiological conditions.

4. The polypeptide particles of claim 3, wherein the stabilizing agent is selected from trehalose and methyl-mannopyranoside.

5. The polypeptide particles of any one of claims 1 to 4, wherein the stabilizing agent is included in the polypeptide particles at a wt/wt ratio of stabilizing agent to polypeptide that ranges between 0.1/1 to 1/1.

6. The polypeptide particles of any one of claims 1 to 4, wherein the stabilizing agent is included in the polypeptide particles at a wt/wt ratio of stabilizing agent to polypeptide that ranges between 0.1/1 to 0.5/1.

7. The polypeptide particles of any one of claims 1 to 4, wherein the stabilizing agent is included in the polypeptide particles at a wt/wt ratio of stabilizing agent to polypeptide that ranges between 0.1/1 to 0.25/1.

8. The polypeptide particles of claim 1, wherein the stabilizing agent comprises a metal ion derived from a divalent metal ion salt.

9. The polypeptide particles of claim 8, wherein the divalent metal ion salt is selected from the group consisting of CaCl₂, MgCl₂, and ZnCl₂.

10. The polypeptide particles of any preceding claim, wherein the stabilizing agent comprises a metal ion and the molar ratio of the stabilizing agent to the polypeptide included in the polypeptide particles ranges from 1/1 to 10/1.

11. The polypeptide particles of any preceding claim, wherein the stabilizing agent comprises a metal ion and the molar ratio of the stabilizing agent to the polypeptide included in the polypeptide particles ranges from 2/1 to 6/1.

12. The polypeptide particles of any preceding claim, wherein the stabilizing agent comprises a metal ion and the molar ratio of the stabilizing agent to the polypeptide included in the polypeptide particles is about 4/1.

13. The polypeptide particles of claim 1, wherein the stabilizing agent is selected from trehalose and methyl-mannopyranoside, and wherein the wt/wt ratio of stabilizing agent to pituitary adenylate cyclase polypeptide included in the polypeptide particles is about f 0.55/1.

14. The polypeptide particles of claim 1, wherein the stabilizing agent is selected from Ca²⁺, Mg²⁺, and Zn²⁺, and wherein the molar ratio of metal ion to pituitary adenylate cyclase polypeptide included in the polypeptide particles is about 4/1.

15. The polypeptide particles of any preceding claim which are formulated to exhibit a reconstitution pH between pH 2 and pH 4.

## Patentansprüche

1. Stabilisierte Polypeptidteilchen, umfassend ein Hypophysenadenylatcyclasepolypeptid und ein Stabilisierungsmittel, ausgewählt aus der Gruppe, bestehend aus Metallionen und unter sauren Bedingungen bei Temperaturen bis zu oder über physiologischen Bedingungen stabilen Zuckern, wobei die Polypeptidteilchen derart formuliert sind, dass sie einen sauren Neubildungs-pH-Wert aufweisen.

2. Polypeptidteilchen nach Anspruch 1, wobei die Polypeptidteilchen ein Hypophysenadenylatcyclasepolypeptid, ein Metallion und einen unter sauren Bedingungen bei Temperaturen bis zu oder über physiologischen Bedingungen stabilen Zucker umfassen.

3. Polypeptidteilchen nach Anspruch 1 oder 2, wobei das Stabilisierungsmittel ausgewählt ist aus unter sauren Bedingungen bei Temperaturen bis zu oder über physiologischen Bedingungen stabilen Disacchariden und Monosacchariden.

4. Polypeptidteilchen nach Anspruch 3, wobei das Stabilisierungsmittel ausgewählt ist aus Trehalose und Methylmannopyranosid.

5. Polypeptidteilchen nach einem der Ansprüche 1 bis 4, wobei das Stabilisierungsmittel mit einem Gew.-. Verhältnis von Stabilisierungsmittel zu Polypeptid, das im Bereich zwischen 0,1/1 bis 1/1 liegt, in den Polypeptidteilchen eingeschlossen ist.

6. Polypeptidteilchen nach einem der Ansprüche 1 bis 4, wobei das Stabilisierungsmittel mit einem Gew.-Verhältnis von Stabilisierungsmittel zu Polypeptid, das im Bereich zwischen 0,1/1 bis 0,5/1 liegt, in den Polypeptidteilchen eingeschlossen ist.

7. Polypeptidteilchen nach einem der Ansprüche 1 bis 4, wobei das Stabilisierungsmittel mit einem Gew.-Verhältnis von Stabilisierungsmittel zu Polypeptid, das im Bereich zwischen 0,1/1 bis 0,25/1 liegt, in den Polypeptidteilchen eingeschlossen ist.

8. Polypeptidteilchen nach Anspruch 1, wobei das Stabilisierungsmittel ein von einem zweiwertigen Metallionensalz abgeleitetes Metallion umfasst.

9. Polypeptidteilchen nach Anspruch 8, wobei das zweiwertige Metallionensalz ausgewählt ist aus der Gruppe, bestehend aus CaCl₂, MgCl₂ und ZnCl₂.

10. Polypeptidteilchen nach einem der vorangehenden Ansprüche, wobei das Stabilisierungsmittel ein Metallion umfasst und das Molverhältnis des Stabilisierungsmittels zu dem in den Polypeptidteilchen eingeschlossenen Polypeptid im Bereich von 1/1 bis 10/1 liegt.

11. Polypeptidteilchen nach einem der vorangehenden Ansprüche, wobei das Stabilisierungsmittel ein Metallion umfasst und das Molverhältnis des Stabilisierungsmittels zu dem in den Polypeptidteilchen eingeschlossenen Polypeptid im Bereich von 2/1 bis 6/1 liegt.

12. Polypeptidteilchen nach einem der vorangehenden Ansprüche, wobei das Stabilisierungsmittel ein Metallion umfasst und das Molverhältnis des Stabilisierungsmittels zu dem in den Polypeptidteilchen eingeschlossenen Polypeptid etwa 4/1 beträgt.

13. Polypeptidteilchen nach Anspruch 1, wobei das Stabilisierungsmittel ausgewählt ist aus Trehalose und Methylmannopyranosid, und wobei das Gew.-Verhältnis von Stabilisierungsmittel zu in den Polypeptidteilchen eingeschlossenem Hypophysenadenylatcyclasepolypeptid etwa 0,55/1 beträgt.

14. Polypeptidteilchen nach Anspruch 1, wobei das Stabilisierungsmittel ausgewählt ist aus Ca²⁺, Mg²⁺ und Zn²⁺, und wobei das Molverhältnis von Metallion zu in den Polypeptidteilchen eingeschlossenem Hypophysenadenylatcyclasepolypeptid etwa 4/1 beträgt.

15. Polypeptidteilchen nach einem der vorangehenden Ansprüche, die derart formuliert sind, dass sie einen Neubildungs-pH-Wert zwischen pH 2 und pH4 aufweisen.

## Revendications

1. - Particules de polypeptide stabilisées comprenant un polypeptide adénylate cyclase pituitaire et un agent de stabilisation choisi dans le groupe constitué par les ions métalliques et les sucres qui sont stables dans des conditions acides à des températures allant jusqu'à ou dépassant les conditions physiologiques, les particules de polypeptide étant formulées pour présenter un pH de reconstitution acide.

2. - Particules de polypeptide selon la revendication 1, dans lesquelles les particules de polypeptide comprennent un polypeptide adénylate cyclase pituitaire, un ion métallique et un sucre qui est stable à un pH acide à des températures allant jusqu'à et dépassant les conditions physiologiques.

3. - Particules de polypeptide selon l'une de revendications 1 ou 2, dans lesquelles l'agent de stabilisation est choisi parmi les disaccharides et les monosaccharides qui sont stables dans des conditions acides à des températures allant jusqu'à et dépassant les conditions physiologiques.

4. - Particules de polypeptide selon la revendication 3, dans lesquelles l'agent de stabilisation est choisi parmi le tréhalose et le méthyl-mannopyranoside.

5. - Particules de polypeptide selon l'une quelconque des revendications 1 à 4, dans lesquelles l'agent de stabilisation est inclus dans les particules de polypeptide à un rapport p/p d'agent de stabilisation au polypeptide qui se situe dans la plage entre 0,1/1 et 1/1.

6. - Particules de polypeptide selon l'une quelconque des revendications 1 à 4, dans lesquelles l'agent de stabilisation est inclus dans les particules de polypeptide à un rapport p/p d'agent de stabilisation au polypeptide qui se situe dans la plage entre 0,1/1 et 0,5/1.

7. - Particules de polypeptide selon l'une quelconque des revendications 1 à 4, dans lesquelles l'agent de stabilisation est inclus dans les particules de polypeptide :à un rapport p/p d'agent de stabilisation au polypeptide :qui se situe dans la plage entre 0,1/1 et 0,25/1.

8. - Particules de polypeptide selon la revendication 1, dans lesquelles l'agent de stabilisation comprend un ion métallique issu d'un sel d'ion métallique divalent.

9. - Particules de polypeptide selon la revendication 8, dans lesquelles le sel d'ion métallique divalent est choisi dans le groupe consistué par CaCl₂, MgCl₂ et ZnCl₂.

10. - Particules de polypeptide selon l'une quelconque des revendications précédentes, dans lesquelles l'agent de stabilisation comprend un ion métallique et le rapport molaire de l'agent de stabilisation au polypeptide qui est inclus dans les particules de polypeptide se situe dans la plage de 1/1 à 10/1.

11. - Particules de polypeptide selon l'une quelconque des revendications précédentes, dans lesquelles l'agent de stabilisation comprend un ion métallique et le rapport molaire de l'agent de stabilisation au polypeptide qui est inclus dans les particules de polypeptide se situe dans la plage de 2/1 à 6/1.

12. - Particules de polypeptide selon l'une quelconque des revendications précédentes, dans lesquelles l'agent de stabilisation comprend un ion métallique, et le rapport molaire de l'agent de stabilisation au polypeptide qui est inclus dans les particules de polypeptide est d'environ 4/1.

13. - Particules de polypeptide selon la revendication 1, dans lesquelles l'agent de stabilisation est choisi parmi le tréhalose et le méthyl-mannopyranoside, et dans lesquelles le rapport p/p d'agent de stabilisation au polypeptide adénylate cyclase pituitaire qui est inclus dans les particules de polypeptide est d'environ 0,55/1.

14. - Particules de polypeptide selon la revendication 1, dans lesquelles l'agent de stabilisation est choisi parmi Ca²⁺, Mg²⁺ et Zn²⁺, et dans lesquelles le rapport molaire de l'ion métallique au polypeptide adénylate cyclase pituitaire qui est inclus dans les particules de polypeptide est d'environ 4/1.

15. - Particules de polypeptide selon l'une quelconque des revendications précédentes, qui sont formulées pour présenter un pH de reconstitution entre pH 2 et pH 4.
